# EUROPEAN PATENT SPECIFICATION

(11) **EP 0 536 187 B1**
(45) Date of publication and mention of the grant of the patent: **21.09.1994**
(21) Application number: 91911126.0
(22) Date of filing: 06.06.1991
(51) Int. Cl.: A61B 5/00, A61B 5/14, G01N 21/35

(54) **METHOD AND DEVICE FOR IN VIVO MEASURING BLOOD SUGAR LEVELS**
VERFAHREN UND GERÄT ZUR MESSUNG DES BLUT-GLUKOSE-GEHALTES IN VIVO
PROCEDE ET DISPOSITIF POUR LA MESURE IN VIVO DU TAUX DE GLYCEMIE

(30) Priority: 06.06.1990 AU 489/90; 22.01.1991 AU 4310/91
(43) Date of publication of application: 14.04.1993
(73) Proprietor: NOVO NORDISK A/S, 2880 Bagsvaerd (DK)
(72) Inventor: CLIFT, Vaughan, DK-2920 Charlottenlund (DK)
(74) Representative: Hansen, Einar Tronier
(86) International application number: DK9100152
(87) International publication number: WO9118548

(56) References cited:
- US-A- 4 427 889
- US-A- 4 509 522
- US-A- 4 655 225
- US-A- 4 882 492

## Description

The present invention concerns a method and a device for measuring in vivo blood sugar levels.

Continuous maintanance of normal blood sugar levels is important in avoiding the deterioration of a number of body organs. For example it is considered a prerequisite in prevention of the progression of diabetic microangiopathy in the kidney, the retina, and in autonomic and peripheral nerves. However, under normal conditions for managing a diabetic condition it is virtually impossible to maintain blood sugar concentrations continuously at normal levels. This is largely because the uptake of carbohydrates and conversion to glucose in the blood is not predictable. Blood sugar levels are normally determined by regular in vitro measurements and the quantity of insulin required to maintain normal blood sugar levels is based on predictions of glucose uptake and consumption during the period between measurements. Thus for example if diabetics find themselves in the position of engaging in unpredicted exercise especially after an injection of insulin, they can all too readily become comatose. Diabetes is hence very difficult to manage in children and adults. Also in the unwell or premature neonate reserves of glucose are limited and control mechanisms immature predisposing them to dangerously low blood sugar levels.

At present blood sugar levels are normally determined by regular in vitro measurements requiring puncturing the skin, taking away a blood sample and using an in vitro method which usually takes two minutes. This induces considerable restraint on taking blood sugar measurements. These constraints on measuring blood sugar levels and poor predictability of blood glucose levels have significant impact on the lifestyle of a diabetic patient and the well being of any seriously ill patient.

As a result of these difficulties in managing a diabetic condition there is a long standing need for a device that is capable of continuously measuring in vivo blood sugar levels. Such a device could be used for controlling the injection of insulin into the blood in response to increases in blood sugar levels. Such a device is known as an artificial pancreas. Attempts have been made to combine in vivo determination of blood sugar levels with an insulin pump controlled by a micro-computer. However, these have been a failure because the method of continuously monitoring blood sugar levels was not reliable. Attempts have been made to make continuous blood sugar measurement in vivo using presently available enzyme techniques such as glucose oxidase reduced onto an electrode. These devices comprise in general the coating of an anode with an enzyme specific for glucose, covering this with a glucose permeable membrane and measuring a small potential difference, or current, between this electrode inserted into a subcutaneous layer with another electrode. However, the method has proved unreliable because the currents and/or potentials are minute and subject to electrical noise, the membranes become coated by the body preventing the diffusion of glucose into the electrode and small molecules like uric acid, enzymes and other factors destroy the enzyme.

Optical methods have also been previously suggested for measuring the glucose molecule using its physical properties such as refractive index, ability to rotate the plane of polarised light and absorption in the infra-red spectrum, especially the near infra-red. For instance, in published Canadian Patent No. 1,247,397 there is disclosed an I.R. absorption method using discrete frequencies of near infra-red light and light transmitted or diffusely reflected from the irradiated tissue. The difficulty with such a method is discussed below.

Infrared spectroscopy has been proposed as an alternative to electrochemical techniques for continuously monitoring in vivo blood sugar levels. The problems associated with such a proposal are described in an article published in the International Journal of Artificial Organs, Vol. 12, No. 2 1988, pages 129 to 135 "Artificial Pancreas Blood Glucose Measured by Infrared Spectroscopy" by H. Zeller, P. Novak and R. Landgraf. The principal difficulty concerns the strong background absorption spectra of water and other blood constituents which make distinguishing the "fingerprint" of glucose difficult. This absorption is so strong as to effectively prevent any method using mid to far I.R. light transmitting the light through any volume of tissue.

Another difficulty in near I.R. is that the technique requires the maintenance of a fixed path length of sample through which the light passes. Also, the varying concentrations of the other components of blood which also absorb I.R. light produce significant error in measurement.

Until now research had concentrated on infra-red spectroscopy or photometric methods in the near infrared as wavelengths of light in this region are capable of penetrating through flesh. However, wavelengths beyond this range have the advantage that absorption by the glucose molecule is stronger and more sharply delineated permitting it to be more readily differentiated from the other components of blood.

Attempts to use the near infra-red have largely been unsuccessful because of the limited absorption by the markedly attenuated glucose overtones in this region and the overlying spectra of numerous molecules including water. Also the systems previously described have had to maintain a fixed path length.

From US-A-4 882 492 a method for measuring the absorption in the infrared band ranging from 900nm to 1800nm or from 1800nm to 3400nm is known. By this method it is measured how the spectrum is modified when infrared light is reflected from the tissue. The absorptions at a glucose measuring wavelength and at a reference wavelength for glucose are determined and a correction for the reference wavelength measurement is made to compensate for any glucose dependence at the reference wavelength.

Other methods such as are disclosed in Swiss patent CH 612,271 have described the use of A.T.R. (Attenuated Total Reflection) prisms and the use of spectroscopy by means of a Fourier Transform Infra-red Spectroscope. These methods have been made inaccurate by the inability of such a method to accurately correct for the varied concentrations of other components of blood, for example, varying haemoglobin and fat, whose spectra overlay that of glucose (see also Clinical Chemistry Vol 35, No. 9 1854-1856). The light must enter and exit the prism at a precise angle and a fluid being examined must cover a constant surface area. These requirements as well as other variables such as temperature of the prism, make the application to a patient difficult and remote sensing, such as bed side monitoring, impossible. Also the "evanescent" wave produced by these A.T.R. systems penetrates less than 1 micron into the surrounding fluid making them impossible to use across a membrane or through skin for measuring blood sugar.

Methods have also been described which use such sources as CO₂ lasers as a source of discrete frequencies absorbed by the glucose molecule. These methods too cannot compensate for the varying concentrations of protein and fat and their effect on accuracy. They have the added disadvantage of requiring expensive and highly specialized bulky equipment and present some risk of burning the tissue being assayed.

It is, therefore, the object of the invention to provide a method for in vivo measurement of the concentration of glucose in the blood by which method these difficulties are overcome and the measurement may be made using an inexpensive and portable system despite the varying concentrations of interfering substances such as other blood constituents including protein and fat.

This is obtained by a method according to claim 1
Known methods have concentrated on infrared spectroscopy in the near range from 0.700 to 1.5*»*m as these wavelengths are capable of penetrating skin. Infrared spectroscopy in the far infrared range has been ignored as it has difficulty penetrating the skin and the weak measurement signals are heavily disturbed by absorption in the other blood constituents, water, protein and fat. However, the glucose molecule is more readily differentiated from the other components of the blood by its absorption spectrum in the far infrared range especially in the range 9-10*»*m.

By the method according to the invention, profit is taken from the clearer "finger prints" of glucose in the far infrared range and the "noise" caused by the absorption in other blood components is compensated for by simultaneously measuring the concentrations of these components with separate reference sample wavelengths and compensating for the error in measurement these produce using experimentally derived constants.

A similar system has been used successfully in a commercial device called a Foos Milko-scan 104 A/B for the meaurement of lactose in samples of dairy products (see US patent 931621). This system analyses milk using transmission through a sample in a test cuvette and measures the light absorbed using a thermopile light detector. Such a system is of course not applicable to in vivo measurement of glucose but serves to illustrate the efficacy of the principle.

The light may be transmitted to the tissue beneath the outer layer of skin and the absorption be measured by attenuated total reflection. By this invasive method and measurement the difficulty of the longwave infrared light, not penetrating the skin, is overcome, but the light may also non-invasively be transmitted to the outer side of the skin and the absorption may be measured by detecting the heat generated in the tissue, both these methods may be applied non-invasively to areas where the tissue is not covered by thick layers of epithelium such as inside the mouth.

The light transmitted to the tissue may be modulated and using different modulation frequencies for the different wavelengths of light used for measuring the concentration of different components the different pairs of wavelengths may be discriminated.

If, further, the different modulation frequencies are not harmonic the respective pairs of measuring and reference wavelengths may be discriminated even if they are transmitted simultaneously through the same transmission channel.

If the modulation of the measuring wavelength and the modulation of the reference wavelength is not in phase these measurements performed by each of these wavelengths may also be discriminated. By this method problems such as changing temperature or exposed surface areas may be overcome as both wavelengths, sample and reference, are exposed to the same conditions.

The modulation may be provided by chopping the light.

The results of the measurements may appear as computable electric signals, and the correction and interpretation of the measured values may be made by calculation performed on the electric signals.

The substance, the concentration of which is measured, is preferably glucose, but the method may also be used for measuring the concentration of CO₂ or ethanol in the blood, or the product of enzymic reaction of glucose.

The sample wavelength and the reference wavelength used are preferably for glucose 9.5 ±0.5*»*m and 7.7 ±0.5 *»*m, respectively, or 3.47 ±0.5 *»*m and 2.96 ±0.5 *»*m, respectively, for fat 5.74 ±0.5 *»*m and 5.58 ±0.5 *»*m, respectively, or 3.513 ±0.5 *»*m and 3.57 ±0.5 *»*m, respectively, for protein 6.5 ±0.5 *»*m and 6.7 ±0.5 *»*m, respectively.

The invention also comprises an apparatus for carrying out the method, which apparatus is defined in claim 17.

The light aggregate may comprise a wide-banded light source and a number of filters each transmitting one of the selected narrow bands, or it may comprise a number of surface emitting light emitting diodes each emitting infrared light of a chosen wavelength.

The light aggregate may further comprise means for focusing the light on an optical coupling means for the light transmitting means.

Further the light aggregate may comprise chopper means for chopping at preset chopping frequences the light wave bands transmitted from the light aggregate. The chopper means may be mechanical in the shape of a rotating diaphragm with cut-outs, or when the light aggregate comprises a number of light emitting diodes or semi-conductor thermal source, an electronic circuit controlling the energization of the light diodes or semi-conductor thermal sources.

The detector may in its invasive form comprise an optical device which in its simplest form consists of an input optical fibre transmitting light from the light aggregate, a gap filledwith tissue fluid or blood across which light passes and the light transmitted passing through an output fibre to a light detector.

The detector may in its invasive form comprise an optical needle probe ATR all having an input fibre and an output fibre and at the output fibre a light detector providing an output signal representing by an electrical signal the light absorption measured. The sensitivity of such an ATR cell may be enhanced by the addition of a thin metal coating to the outside of the cell such that it acts as a surface plasma resonance device.

The detector may have an overlying jacket of a glucose permeable membrane with a gap for fluid to collect to decrease the concentration of interfering substances further. The detector may also in its invasive form use the glucose permeable membrane jacketed ATR needle in conjunction with an enzyme and the method be used to measure glucose indirectly by means of measuring a product of enzymic reaction the infra-red absorption of which is measured in the same way as for glucose.

For non-invasive measurement the detector may comprise a pair of chambers having an open side placed against the skin on the measuring spot, and a pressure transducer measuring the pressure difference between the chambers and representing this differential pressure by an electric signal. The one chamber has at its side opposite the open side an inlet for infrared light radiation transmitted from the light aggregate. This construction makes it possible to detect the pressure produced in the chamber lying over the tissue which receives infrared light radiation when the absorption of this radiation is converted into heat in the tissue. Commonly made noises, e.g. those due to the pulse movement or due to pressure changes caused by common temperature variations in the skin, will automatically be rejected.

Such a method for use with ultra violet and visible light has been used for measurement of skin pigment and was published by Drs Poulet and Chambron in The Journal of Medical and Biological Engineering and Computing, 1985, 23, 585-588. A similar method is described in European patent application No. 282,234. However, in the method we are using, light from the mid to far infra-red region is used which has not been described previously for an in vivo application. Also, in such previously described methods there is no method to solve the problem of interfering substances producing error in a quantitative assay, a problem overcome by this method.

Using such a two chamber detector receiving infrared light radiation in both its chambers makes it possible to measure the response produced by the light absorption of a sample wavelength against the response produced by the corresponding reference wavelength.

To compensate for noise caused by vibrations in the direction perpendicular to the diaphragm of the pressure transducer between the chambers a further transducer may be added to each chamber at the ends thereof and with their diaphragms in planes parallel with the diaphragm of the transducer between the chambers.

Another important source of disturbance is sweat secreted into the chambers. This may be eliminated by letting each chamber having an inlet for infrared radiation, communicate with a compartment containing a desiccant. In this way the water may be removed whereas the pressure provided in the chamber is maintained. This may also be achieved by means of a rigid but water permeable membrane to transport the water to the outside.

In the following, the invention will be explained with reference to the drawings, wherein
- Fig. 1: schematically shows a light aggregate for providing chopped infrared radiation of chosen wavelengths,
- Fig. 2: shows a diaphragm used for providing different chopping frequencies,
- Fig. 3: schematically shows a side elevation view of an optical fibre detector with ATR needle probe,
- Fig. 4: shows details of an ATR needle probe with an internal infusion canal,
- Fig. 5: shows details of an ATR needle probe attached to an infusion needle,
- Fig. 6: shows an optical fibre detector with an ATR needle probe with separate input fibres for each wavelength,
- Fig. 7: schematically shows an ATR needle probe inserted through the skin,
- Fig. 8: shows a detector detecting the heat response of the tissue beneath the skin when infrared radiation is transmitted to the skin,
- Fig. 9: shows another embodiment of a heat response detector,
- Fig. 10: shows a further development of the heat response detector in Fig. 9,
- Fig. 11: shows an electric coupling adding the outputs from the transducers of the detector in Fig. 10,
- Fig. 12: shows a heat response detector with an infrared radiation source in the form of emitting diodes carried on the detector,
- Fig. 13: shows seen from the bottom a double detector of the kind shown in Fig. 9,
- Fig. 14: schematically shows a view from the bottom of a triplicated detector of the kind shown in Fig. 9.
- Fig. 15: shows another embodiment of the detector with a desiccant chamber,
- Fig. 16: shows another embodiment of the detector with a water permeable membrane,
- Fig. 17: shows a further development of the ATR needle probe detector with a glucose permeable membrane, and
- Fig. 18: shows a detector detecting the transmission of light through a sample.

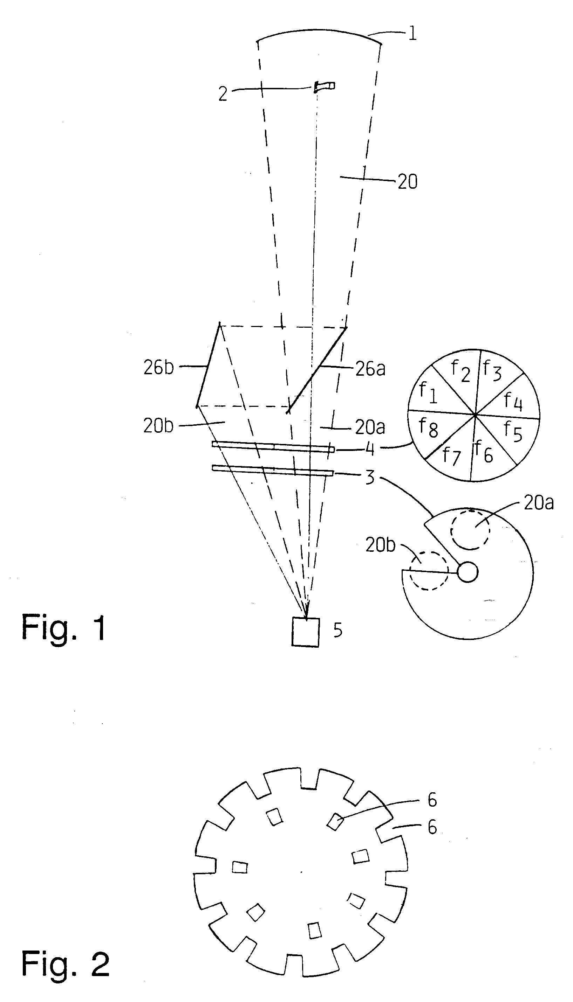

A light aggregate providing infrared light in selected wavelength bands is shown schematically in Fig. 1 wherein 2 is a source of wide-banded light and 1 is a concave mirror concentrating the light from the source 2 into a beam 20 of light which is passed through a beam splitting device comprising a semi-transparent reflecting surface 26a and a totally reflecting surface 26b. The two beams 20a,20b of light provided in this way are transmitted through two different areas of a rotable filter disc 4 which areas form filters f₁-f₈ for measuring wave bands and a reference wave bands, respectively.

In the path of the two beams 20a and 20b a rotary diaphragm 3 is placed having its centre between the two beams and having a cut out sector. By rotating this diaphragm 3 the two light beams are chopped with the same frequency defined by the rotation speed of the diaphragm 3 and as indicated by the dotted circles the beams 20a and 20b are positioned such that the chopping of the respective beams will be out of phase. The aggregate is so designed that the two chopped beams are directed towards an optical coupling means 5 for coupling to the transmitting means transmitting the light to a sensor. The transmitting means may be an optical fibre or the transmission may take place directly through the air.

Fig. 2 shows another embodiment of the diaphragm 3. This diaphragm is designed to be rotated about a centre placed at one side of the two beams and it has two sets of apertures 6 each set being placed on a circle concentric with the diaphragm, the numbers of apertures and the radii of the circles being different for the respective sets. The difference of the radii is equal to the distance between the two beams of infrared light. In this way the two light beams may be chopped at different frequencies.

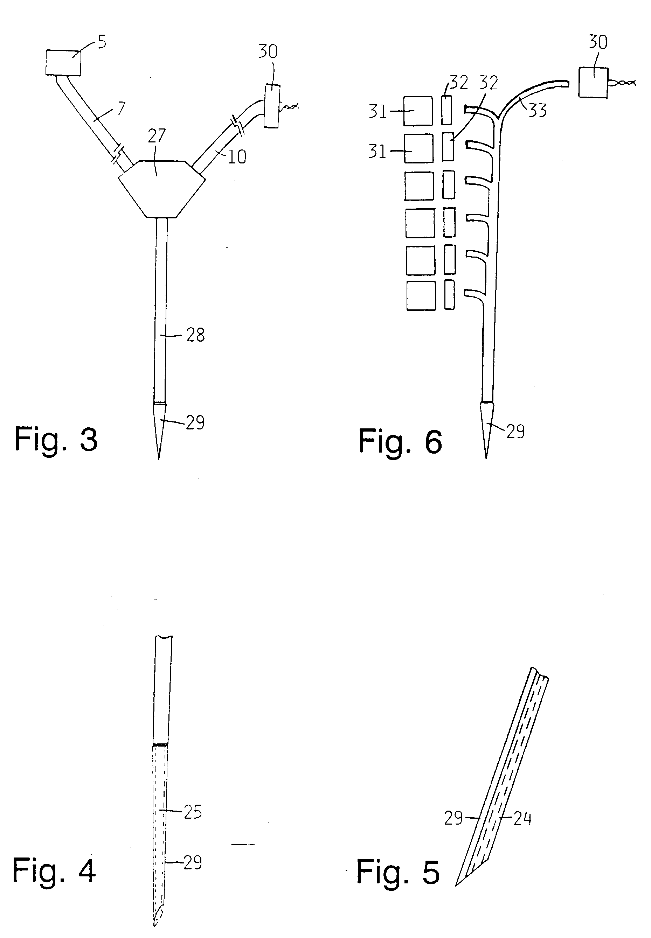

Fig. 3 schematically shows a detection device based on the attenuated total reflection (ATR) principle. From the coupling means 5 light is transmitted through an optical fibre 7, or fibre bundle through an optical device 27 directing the light from the fibre 7 through another optical fibre 28 to a single ended ATR needle probe 29. The reflected light passes through the fibre 28 and is in the device 27 directed through the fibre 10 to a detector 30. As the active area of the ATR needle is the outer surface a further embodiment of this device includes the ATR needle 29 having a central canal as illustrated in Fig. 4. Fig. 5 shows a similar embodiment with the ATR needle 29 attached to a metal needle 24 for infusion of fluids.

In Fig. 6 an input fibre to the ATR-needle 29 comprises a number of fibres each connected to its individual semi-conductor thermal device 31. Monochromatic IR filters 32 between each thermal device 31 and its corresponding fibre allow each fibre in the bundle to carry a specific light wave band. A specific light wave band for the fibre may also be produced using a single thermal device 31 and a filter wheel. In another embodiment not shown the devices 31 may be narrow band light emitting diodes each emitting infrared light in a chosen band. In this case no monochromatic IR filters would be needed.The reflected light passes back up the core fibre of the bundle 33 to the detector 30.

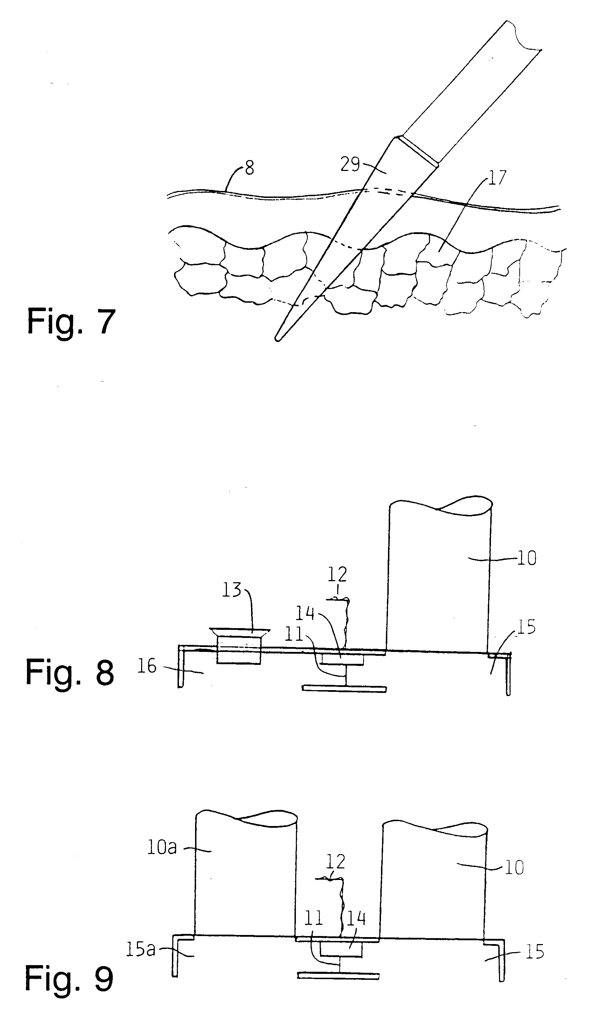

The ATR needle 29 is inserted through the skin 8 into the subcutaneous tissue 17 as shown in Fig. 7. As shown in Figs. 4 and 5, the ATR-needle probe may have a canal for infusion of fluid or it may be attached to a needle for subcutaneous infusion of a fluid, e.g. insulin.

Fig. 8 shows a detector which may be used for non-invasive detection of the absorption of infrared light transmitted to the skin. The detector comprises a sensing chamber 15 and a reference chamber 16 and a differential pressure transducer 14 measuring the pressure difference between the chambers 15 and 16 and giving off an electric signal through leads 12, this signal being representative of the pressure difference. The reference chamber 16 has a screw 13 for adjusting the volume of the response by damping background noise.

Infrared light is transmitted from the light aggregate to the sensing chamber through an optical fibre 10 and will pass across the air space in the sensing chamber and into the skin.

As the beam of radiation passes through the skin into the underlying tissue certain molecules absorb radiation of specific wavelengths and convert it into heat. This heat then diffuses to the surface and heats the air in the chamber producing a pressure wave. The light which is pulsed stimulates cycles of heating and subsequent cooling in the skin which in turn produces pressure waves in the chamber at the same frequency as the pulses of light. This is detected by the pressure transducer 14 and is converted into an electrical signal. The amplitude of the electrical signal is in proportion to the amount of heat produced which is proportional to the number of molecules or the concentration of that substance.

Pressure waves in the detector may be produced by sound waves arising from the body, and are therefore noise, reach both chambers and the pressure changes in both chambers therefore being equal do not move the transducer diaphragm placed between the chambers.

As the heat from the deeper layers will take longer to diffuse to the surface the delay in the signal can be used to relatively select out an area of the deeper layers of skin. In this way the concentration of the blood sugar in the vessels of the dermis can be measured.

In another embodiment shown in Fig. 9 infrared light is transmitted to both of the chambers of the detector, light of the sample wavelength being transmitted to one chamber 15 and light of the reference light wavelength being transmitted to the other 15a. The transducer 14 measures the pressure difference between the two chambers 15 and 15a and the response signal is led to a measuring device by leads 12.

By adjusting the light radiation of the reference and the sample beam such that the pressure difference between the two chambers is low when the concentration of the measured substance in the skin is low much improved sensitivity of the pressure transducer can be obtained by increasing the sensitivity of the pressure difference transducer.

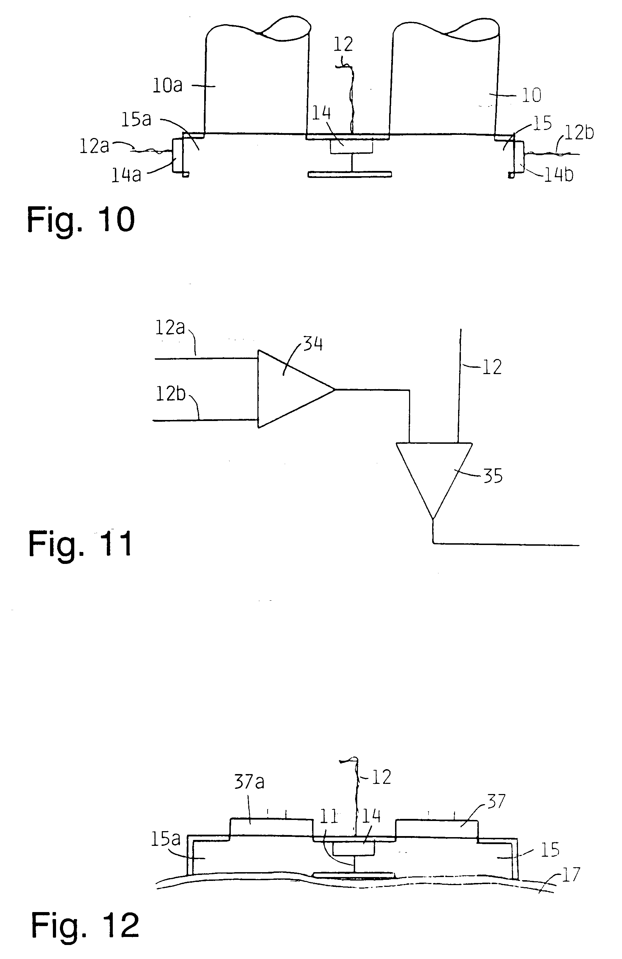

In a further development of the above detector as shown in Fig. 10 two further pressure transducers 14a and 14b are added, at the end of the chamber lying opposite the transducer 14 between the chambers 15 and 15a and with their diaphragms lying in planes parallel with the plane of the diaphragm of the transducer 14. As the two further transducers 14a and 14b face each other their signals are opposite when their diaphragms are relatively moved due to movement of the total device in a direction perpendicular to the planes of the diaphragms.

The signals from the transducers 14a and 14b are amplified and balanced and electrically added to the signal from the differential pressure transducer 14 as shown by the electrical diagram in Fig. 11. One of the signals from leads 12a and 12b is inverted and added to the other in the amplifier 34, and the added signal is added to the differential signal on the leads 12, amplified in an amplifier 35. By so doing the response in the chambers is increased and the noise due to horizontal vibration reduced.

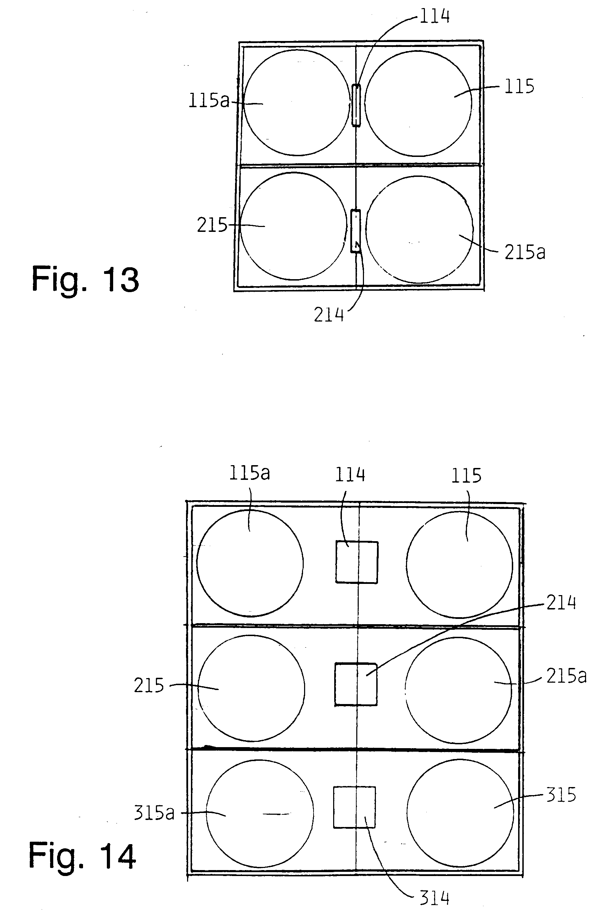

In the above embodiments the light is transmitted from a light aggregate to the detector through optical fibres or other kinds of light transmitting means. In another embodiment shown in Fig. 12 the light is generated in the detector by surface emitting light diodes 37 and 37a emitting infrared light at the sample wavelength and the reference wavelength, respectively. From the light diodes 37 and 37a light passes over the space of the chambers 15 and 15a into the tissue 17. The pressure change produced is measured by the pressure transducer 14 and the response signal is carried by the leads 12 to a measuring and computing device.

The use of light emitting diodes will make the detector more mobile as only electric connections to controlling means and to the measuring and calculating device will be necessary. Less mobile is a detector which further has to be connected to a light aggregate through an optical fibre. Embodiments using a conventional optical transmission will be immobile to an extent making it necessary to bring the measuring object to the device rather than bringing the detector to the sampling spot of the patient.

With the above thermal detectors the relation between the response of the tissue to a sample wavelength and to its reference wavelength may be measured. The response may be measured for all the sample wavelengths and the reference wavelengths in sequence or overlapping in a way making it possible to discriminate the individual relations.

Another way of performing a simultaneous measurement at two wavelengths, e.g. a sample wavelength and a reference wavelength and canceling the effect of "lateral" movement is using a detector comprising a doubling of the device according to Fig. 9. In this way four chambers 115, 115a, 215 and 215a are provided as seen in Fig. 13 showing the double detector seen from the bottom. The two sets of sample and reference wavelengths are transmitted to the respective detector units through optical fibres and the response of the tissue to the two sets of wavelengths is measured by transducers 114 and 214. The detector units being oriented oppositely implies that signals from the two transducers 114 and 214 due to longitudinal vibrations could be added to each other and balanced out.

In the same way the detector of Figs. 9 to 12 may be triplicated to measure the relations of the responses to three pairs of wavelengths that are three pairs of sample wavelengths and reference wavelengths thus decreasing measuring time and eliminating noise from horizontal vibration. Such a triplicated detector is schematically shown seen from the bottom in Fig. 14. The sample and reference chambers have the reference numbers 115, 215, 315 and 115a, 215a, 315a, respectively, infrared light being transmitted to each chamber through optical fibres. Each transmitting fibre carries an individual wavelength.

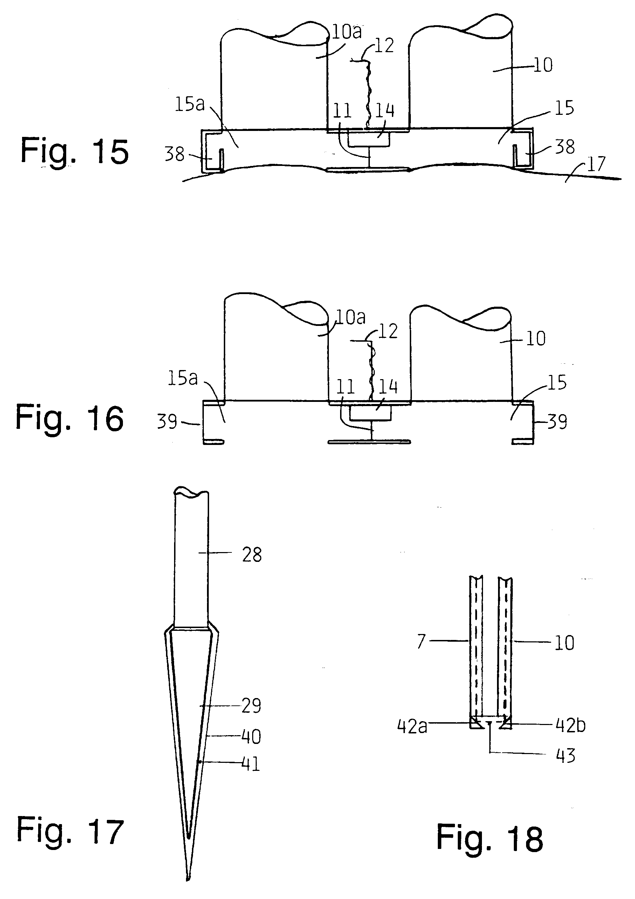

As collection of condensing water especially in a chamber to which infrared radiation is transmitted in a thermal detector of the kind described impairs the measurement precautions should be taken to remove water and water vapour from such chambers. This may be done as sketched in Fig. 15 by providing compartments 38 communicating with the chamber to be dehydrated and place a desiccant in this compartment. Another possibility is as sketched in Fig. 16 to provide the chambers 15, 15a with water permeable membranes 39 between the inner of the chambers 15, 15a, respectively, and the atmosphere.

The efficiency of the ATR needle probe 29 shown in Fig. 3 can be further enhanced by the addition of an extremely thin metal coating to be outside of the ATR needle (not shown) so as to make it act as a surface plasma resonance device. Fig. 17 illustrates a further embodiment of the ATR needle shown in Fig. 3. The probe is jacketed by a glucose permeable membrane 40 with a space below 41 for fluid to collect between the membrane 40 and the ATR needle 29. Furthermore, the membrane may incorporate an enzyme (not shown) and ATR needle detector used to measure a product of that enzymic reaction in the fluid space 41.

Fig. 18 illustrates a further detector whereby the light is transmitted from a coupling device (not shown) down an input optical fibre 7, through a lens (not shown) and reflected by mirror 42a across a gap 43 into which tissue fluid may freely enter and the transmitted light is reflected by mirror 42b into the output optical fibre 10 and to a light detector (not shown).

## Claims

1. A method for in vivo measurement of the concentration of a substance, especially glucose, in the blood despite varying concentrations of interfering components, such as protein and fat, by measuring the absorption of selected wavelengths of infrared light from the range 1 - 40 *»*m transmitted to the tissue of a person whose blood is being tested for its content of the substance, at least one of the wavelengths being selected from the range 3 - 10 *»*m, comprising the following steps
transmitting for each of the substance and the components to be measured two different wavelengths of infrared light, one being a measuring wavelength at which the component or substance being measured shows a specific absorption and the other being a reference wavelength at which the component or substance being measured shows a low absorption,
measuring for each wavelength transmitted the radiation absorbed by the tissue using a detector placed in or on the skin and providing an electric signal which is an expression of the absorbed radiation,
calculating the concentrations of the interfering components, and
calculating the concentration of the substance using the absorption measurement at the substance measuring and reference wavelengths taking into account the interfering absorption caused by the interfering components at the measuring and reference wavelengths for this substance, the interfering absorption being calculated taking into account the measured concentrations of the interfering components and experimentally derived constants.

2. A method according to claim 1, **characterized** in that the light is transmitted to the tissue and the light absorption is measured by attenuated total reflection transcutaneously, i.e. invasively or non-invasively.

3. A method according to claim 1, **characterized** in that the light is transmitted to the outer side of the skin and the absorption is measured by detecting the heat generated in the tissue beneath the skin.

4. A method according to any of the preceding claims, **characterized** in that the light transmitted to the tissue is modulated.

5. A method according to claim 4, **characterized** in that different modulation frequencies are used for the light used for measuring the concentration of different components and substances.

6. A method according to claim 5, **characterized** in that the different modulation frequencies are not harmonic.

7. A method according to any of the claims 4 to 6, **characterized** in that the modulation of the measuring wavelength and the modulation of the reference wavelength are not in phase.

8. A method according to any of the claims 4 - 7, **characterized** in that the modulation is performed by chopping the light.

9. A method according to any of the preceding claims, **characterized** in that the substance the concentration of which is measured is glucose.

10. A method according to any of the preceding claims, **characterized** in that the substance the concentration of which is measured is CO2.

11. A method according to any of the preceding claims, **characterized** in that the substance the concentration of which is measured is ethanol.

12. A method according to any of the preceding claims, **characterized** in that the sample wavelength and the reference wavelength used for glucose are 9.5 ±0.5 *»*m and 7.7 ±0.5 *»*m, respectively.

13. A method according to any of the claims 1-11, **characterized** in that the sample wavelength and the reference wavelength used for glucose are 3.47 ±0.5 *»*m and 2.96 ±0.5 *»*m, respectively.

14. A method according to any of the preceding claims, **charaterized** in that the sample wavelength and the reference wavelength for fat are 5.74 ±0.5 *»*m and 5.58 ±0.5 *»*m, respectively.

15. A method according to any of the claims 1-13, **characterized** in that the sample wavelength and the reference wavelength for fat are 3.513 ±0.5 *»*m and 3.57 ±0.5 *»*m, respectively.

16. A method according to any of the preceding claims, **charaterized** in that the sample wavelength and the reference wavelength for protein are 6.5 ±0.5 *»*m 5 and 6.7 ±0.5 *»*m, respectively.

17. An apparatus for in vivo measurement of the concentration of a substance, especially glucose, in the blood despite varying concentrations of interfering components, such as protein and fat, by measuring the absorption of selected wavelengths of infrared light from the range 1 - 40 *»*m transmitted to the tissue of a person whose blood is being tested for its content of the substance, at least one of the wavelengths being selected from the range 3 - 10 *»*m, comprising
means for transmitting for each of the substance and the components to be measured two different wavelengths of infrared light, one being a measuring wavelength at which the component or substance being measured shows a specific absorption and the other being a reference wavelength at which the component or substance being measured shows a low absorption,
means for measuring for each wavelength transmitted the radiation absorbed by the tissue using a detector placed in or on the skin and providing an electric signal which is an expression of the absorbed radiation,
a computer for calculating the concentrations of the interfering components, and for
calculating the concentration of the substance using the absorption measurements at the substance measuring and reference wavelengths taking into account the interfering absorption caused by the interfering components at the measuring and reference wavelengths for this substance, the interfering absorption being calculated taking into account the measured concentrations of the interfering components and experimentally derived constants.

18. An apparatus according to claim 17, **characterized** in that the light aggregate comprises a wide-banded light source and a number of filters each transmitting one of the selected narrow light bands.

19. An apparatus according to claim 17, **characterized** in that the light aggregate comprises a number of surface emitting light diodes each emitting infrared light of a chosen wavelength.

20. An apparatus according to claim 17, 18 or 19, **characterized** in that the light aggregate comprises means for focussing the light on an optical coupling means for the light transmitting means.

21. An apparatus according to any of the claims 17 - 20, **characterized** in that the light aggregate comprises chopper means for chopping at preset chopping frequencies the light wave bands transmitted from the light aggregate.

22. An apparatus according to claim 21, **characterized** in that the chopper means is a rotating diaphragm with cut-outs.

23. An apparatus according to claims 19 and 21, **characterized** in that the chopper means is an electronic circuit controlling the energization of the light diodes or semiconductor sources.

24. An apparatus according to any of the preceding claims, **characterized** in that the detector comprises an optical needle probe ATR cell having an input fibre and an output fibre, and at the output fibre a light detector providing an output signal, representing by an electric signal the light absorption measured.

25. An apparatus according to any of the claims 17 - 23, **characterized** in that the detector comprises a pair of chambers having an open side placed against the skin at the measuring spot, and a pressure transducer measuring the pressure difference between the chambers representing this diffential pressure by an electric signal.

26. An apparatus according to claim 25, **characterized** in that the one chamber at its side opposite the open side has an inlet for infrared radiation transmitted from the light aggregate.

27. An apparatus according to claim 25, **characterized** in that both chambers have at their sides opposite the open sides inlets for infrared radiation transmitted from the light aggregate.

28. An apparatus according to claim 27, **characterized** in that a further transducer is added to each chamber at the ends thereof and with its diaphragm lying in a plane parallel with the plane of the diaphragm of the transducer between the chambers.

29. An apparatus according to any of the claims 25 - 28, **characterized** in that each chamber having an inlet for infrared radiation communicates with a compartment containing a desiccant.

30. An apparatus according to any of the claims 25 - 28, **characterized** in that each chamber having an inlet for infrared radiation has a wall with a rigid water permeable membrane.

31. An apparatus according to claim 24, **characterized** in that the ATR needle probe cell is coated with a metal such that it acts as a surface plasma resonance device.

32. An apparatus according to claim 31, **characterized** in that the metal coated ATR needle probe may be jacketed by a cellulose permeable membrane with a gap between the membrane and the probe.

33. An apparatus according to claim 32, **characterized** in that the membrane may have a glucose specific enzyme or system of enzymes and the ATR probe used to measure the product of enzymic reaction of the glucose molecule.

34. An apparatus according to claims 17-23, **characterized** in that the detector comprises an optical device with input and output optical fibres with an optical element comprising a sample space for a fluid sample and a transmission measurement made of that sample.

## Patentansprüche

1. Verfahren zur in-vivo-Messung der Konzentration einer Substanz, insbesondere Glucose, im Blut trotz variierender Konzentrationen störender Komponenten, wie z.B. Protein und Fett, durch Messung der Absorption ausgewählter Wellenlängen infraroten Lichts im Bereich von 1 bis 40 *»*m, die zum Gewebe einer Person transmittiert werden, deren Blut bezüglich seines Gehalts an der Substanz getestet wird, wobei wenigstens eine der Wellenlängen im Bereich von 3 bis 10 *»*m gewählt ist, das die folgenden Schritte umfaßt:
Transmission von zwei verschiedenen Wellenlängen infraroten Lichts für die zu messende Substanz und jede der Komponenten, wobei eine eine Meßwellenlänge ist, bei der die zu messende Komponente oder Substanz eine besondere Absorption zeigt, und die andere eine Referenzwellenlänge ist, bei der die gemessene Komponente oder Substanz eine geringe Absorption zeigt,
Messung der vom Gewebe absorbierten Strahlung für jede transmittierte Wellenlänge unter Verwendung eines Detektors, der in oder auf der Haut angeordnet ist und ein elektrisches Signal liefert, das ein Ausdruck für die absorbierte Strahlung ist,
Berechnung der Konzentrationen der störenden Komponenten, und
Berechnung der Konzentration der Substanz unter Verwendung der Absorptionsmessung bei der Substanz-Meß- und -Referenzwellenlange unter Berücksichtigung der störenden Absorption, die durch die störenden Komponenten bei den Meß- und Referenzwellenlängen für diese Substanz verursacht wird, wobei die störne Absorption unter Berücksichtigung der gemessenen Konzentrationen der störenden Komponenten und der experimentell erhaltenen Konstanten berechnet wird.

2. Verfahren nach Anspruch 1, dadurch gekennzeichnet, daß das Licht zum Gewebe transmittiert wird und die Lichtabsorption transkutan, d.h. invasiv oder nicht-invasiv, anhand gedämpfter Totalreflexion gemessen wird.

3. Verfahren nach Anspruch 1, dadurch gekennzeichnet, daß das Licht zur Außenseite der Haut transmittiert wird und die Absorption durch Detektion der Wärme, die in dem Gewebe unter der Haut erzeugt wird, gemessen wird.

4. Verfahren nach einem der vorangehenden Ansprüche, dadurch gekennzeichnet, daß das zum Gewebe transmittierte Licht moduliert ist.

5. Verfahren nach Anspruch 4, dadurch gekennzeichnet, daß verschiedene Modulationsfrequenzen für das Licht, das zur Messung der Konzentration verschiedener Komponenten und Substanzen verwendet wird, verwendet werden.

6. Verfahren nach Anspruch 5, dadurch gekennzeichnet, daß die verschiedenen Modulationsfrequenzen nicht harmonisch sind.

7. Verfahren nach einem der Ansprüche 4 bis 6, dadurch gekennzeichnet, daß die Modulation der Meßwellenlänge und die Modulation der Referenzwellenlänge nicht in Phase sind.

8. Verfahren nach einem der Ansprüche 4 bis 7, dadurch gekennzeichnet, daß die Modulation durch Choppen des Lichts ausgeführt wird.

9. Verfahren nach einem der vorangehenden Ansprüche, dadurch gekennzeichnet, daß die Substanz, deren Konzentration gemessen wird, Glucose ist.

10. Verfahren nach einem der vorangehenden Ansprüche, dadurch gekennzeichnet, daß die Substanz, deren Konzentration gemessen wird, CO₂ ist.

11. Verfahren nach einem der vorangehenden Ansprüche, dadurch gekennzeichnet, daß die Substanz, deren Konzentration gemessen wird, Ethanol ist.

12. Verfahren nach einem der vorangehenden Ansprüche, dadurch gekennzeichnet, daß die für Glucose verwendete Meßwellenlänge und die Referenzwellenlänge jeweils 9,5 ±0,5 *»*m und 7,7 ±0,5 »m betragen.

13. Verfahren nach einem der Ansprüche 1 bis 11, dadurch gekennzeichnet, daß die für Glucose verwendete Meßwellenlänge und die Referenzwellenlänge jeweils 3,47 ±0,5 *»*m und 2,96 ±0,5 *»*m betragen.

14. Verfahren nach einem der vorangehenden Ansprüche, dadurch gekennzeichnet, daß die Meßwellenlänge und die Referenzwellenlänge für Fett jeweils 5,74 ±0,5 *»*m und 5,58 ±0,5 *»*m betragen.

15. Verfahren nach einem der Ansprüche 1 bis 13, dadurch gekennzeichnet, daß die Meßwellenlänge und die Referenzwellenlänge für Fett jeweils 3,513 ±0,5 *»*m und 3,57 ±0,5 *»*m betragen.

16. Verfahren nach einem der vorangehenden Ansprüche, dadurch gekennzeichnet, daß die Meßwellenlänge und die Referenzwellenlänge für Protein jeweils 6,5 ±0,5 *»*m und 6,7 ±0,5 *»*m betragen.

17. Vorrichtung zur in-vivo-Messung der Konzentration einer Substanz, insbesondere Glucose, im Blut trotz variierender Konzentrationen störender Komponenten, wie z.B. Protein und Fett, durch Messung der Absorption ausgewählter Wellenlängen infraroten Lichts im Bereich von 1 bis 40 *»*m, die zum Gewebe einer Person transmittiert werden, deren Blut bzgl. seines Gehalts an der Substanz getestet wird, wobei wenigstens eine der Wellenlängen im Bereich von 3 bis 10 *»*m gewählt ist, die umfaßt:
Mittel zur Transmission von zwei verschiedenen Wellenlängen infraroten Lichts für die zu messenden Substanz und jede der Komponenten, wobei eine eine Meßwellenlänge ist, bei der die zu messende Komponente oder Substanz eine besondere Absorption zeigt, und die andere eine Referenzwellenlänge ist, bei der die zu messende Komponente oder Substanz eine geringe Absorption zeigt,
Mittel zum Messen der vom Gewebe absorbierten Strahlung für jede transmittierte Wellenlänge unter Verwendung eines Detektors, der in oder auf der Haut angeordnet ist und ein elektrisches Signal liefert, das ein Ausdruck für die absorbierte Strahlung ist,
ein Computer zur Berechnung der Konzentrationen der störenden Komponenten, und
zur Berechnung der Konzentration der Substanz unter Verwendung der Absorptionsmessungen bei der Substanz-Meß- und -Referenzwellenlänge unter Berücksichtigung der störenden Absorption, die durch die störenden Komponenten bei den Meß- und Referenzwellenlängen für diese Substanz verursacht wird, wobei die störende Absorption unter Berücksichtigung der gemessenen Konzentrationen der störenden Komponenten und der experimentell erhaltenen Konstanten berechnet wird.

18. Vorrichtung nach Anspruch 17, dadurch gekennzeichnet, daß das Lichtaggregat eine breitbandige Lichtquelle und eine Anzahl von Filtern, wobei jedes eines der gewählten engen Lichtbänder transmittiert, umfaßt.

19. Vorrichtung nach Anspruch 17, dadurch gekennzeichnet, daß das Lichtaggregat eine Anzahl von oberflächenemittierenden Leuchtdioden, wobei jede infrarotes Licht einer gewählten Wellenlänge emittiert, umfaßt.

20. Vorrichtung nach Anspruch 17, 18 oder 19, dadurch gekennzeichnet, daß das Lichtaggregat Mittel zur Fokussierung des Lichts auf eine optische Koppeleinrichtung für die Lichttransmissionseinrichtung umfaßt.

21. Vorrichtung nach einem der Ansprüche 17 bis 20, dadurch gekennzeichnet, daß das Lichtaggregat Chop-Mittel zum Choppen der Lichtwellenbänder, die vom Lichtaggregat transmittiert werden, mit vorher festgelegten Chop-Frequenzen umfaßt.

22. Vorrichtung nach Anspruch 21, dadurch gekennzeichnet, daß das Chop-Mittel eine rotierende Membran mit Ausschnitten ist.

23. Vorrichtung nach Anspruch 19 und 21, dadurch gekennzeichnet, daß das Chop-Mittel ein elektronischer Schaltkreis ist, der die Anregung der Leuchtdioden oder Halbleiterquellen steuert.

24. Vorrichtung nach einem der vorangehenden Ansprüche, dadurch gekennzeichnet, daß der Detektor eine optische ATR-Nadelsondenzelle, die eine Einkoppel- und eine Auskoppelfaser hat und an der Auskoppelfaser einen Lichtdetektor umfaßt, der ein Ausgabesignal liefert, das anhand eines elektrischen Signals die gemessene Lichtabsorption darstellt.

25. Vorrichtung nach einem der Ansprüche 17 bis 23, dadurch gekennzeichnet, daß der Detektor ein Paar Kammern, die eine offene Seite haben, die zur Haut auf dem Meßpunkt angeordnet ist, und einen Druckwandler umfaßt, der die Druckdifferenz zwischen den Kammern mißt und diesen Differenzdruck durch ein elektrisches Signal dargestellt.

26. Vorrichtung nach Anspruch 25, dadurch gekennzeichnet, daß die eine Kammer auf ihrer Seite gegenüber der offenen Seite einen Einlaß für infrarote Strahlung hat, die vom Lichtaggregat transmittiert wird.

27. Vorrichtung nach Anspruch 25, dadurch gekennzeichnet, daß beide Kammern an ihren Seiten gegenüber den offenen Seiten Einlässe für infrarote Strahlung haben, die von dem Lichtaggregat transmittiert wird.

28. Vorrichtung nach Anspruch 27, dadurch gekennzeichnet, daß ein weiterer Wandler jeder Kammer an den Enden derselben und mit seiner Membran in einer Ebene parallel zur Ebene der Membran des Aufnehmers zwischen den Kammern hinzugefügt ist.

29. Vorrichtung nach der Ansprüche 25 bis 28, dadurch gekennzeichnet, daß jede Kammer, die einen Einlaß für infrarote Strahlung hat, mit einer Kammer in Verbindung steht, die ein Trockenmittel enthält.

30. Vorrichtung nach einem der Ansprüche 25 bis 28, dadurch gekennzeichnet, daß jede Kammer, die einen Einlaß für infrarote Strahlung hat, eine Wand mit einer starren, wasserdurchlässigen Membran hat.

31. Vorrichtung nach Anspruch 24, dadurch gekennzeichnet, daß die ATR-Nadelsondenzelle mit einem Metall so beschichtet ist, daß sie wie eine Oberflächenplasmaresonanzeinrichtung wirkt.

32. Vorrichtung nach Anspruch 31, dadurch gekennzeichnet, daß die mit dem Metall beschichtete ATR-Nadelsonde von einer zellulosedurchlässigen Membran mit einem Spalt zwischen der Membran und der Sonde eingehüllt sein kann.

33. Vorrichtung nach Anspruch 32, dadurch gekennzeichnet, daß die Membran ein glucosespezifisches Enzym oder Enzymsystem hat und die ATR-Sonde verwendet wird, um das Produkt der Enzymreaktion des Glucose-Moleküls zu messen.

34. Vorrichtung nach Anspruch 17 bis 23, dadurch gekennzeichnet, daß der Detektor eine optische Einrichtung mit optischen Einkoppel- und Auskoppelfasern mit einem optischen Element umfaßt, das einen Probenraum für eine Flüssigkeitsprobe und eine Transmissionsmessung an dieser Probe umfaßt.

## Revendications

1. Procédé pour la mesure in vivo des concentrations d'une substance, notamment du glucose, dans le sang en dépit de concentrations variables de composants perturbateurs tels que les protéines et les lipides, en mesurant l'absorption des longueurs d'ondes sélectionnées de la lumière infrarouge allant de la plage 1- 40 »m transmise au tissu d'une personne dont le sang est analysé pour déterminer sa teneur en la substance, au moins l'une des longueurs d'ondes étant sélectionnée à partir de la plage 3 à 10 »m comprenant les étapes suivantes :
transmission pour chacune des substances et composants à mesurer de deux longueurs d'ondes différentes de lumière infrarouge, chacune étant une longueur d'onde de mesure à laquelle le composant ou la substance à mesurer montre une absorption spécifique et l'autre étant une longueur d'onde de référence à laquelle le composant ou la substance à mesurer montre une faible absorption,
mesure pour chaque longueur d'onde transmise de la radiation absorbée par le tissu en utilisant un détecteur placé dans ou sur la peau et fournissant un signal électrique qui est une expression de la radiation absorbée,
calcul de la concentration des composants d'interférence, et
calcul de la concentration de la substance en utilisant la mesure d'absorption au niveau de la longueur d'onde de référence en prenant en considération l'absorption d'interférence provoquée par le composant d'interférence au niveau des longueurs d'ondes de mesure et de référence pour cette substance, l'absorption d'interférence étant calculée en prenant en considération les concentrations mesurées des composants d'interférence et des constantes dérivées expérimentalement.

2. Procédé selon la revendication 1, caractérisé en ce que la lumière est transmise au tissu et l'absorption de la lumière est mesurée par réflection totale atténuée par voie transcutanée, c'est-à-dire par voie envahissante ou non envahissante.

3. Procédé selon la revendication 1, caractérisé en ce que la lumière est transmise au côté extérieur de la peau et l'absorption est mesurée par détection de la chaleur produite dans le tissu au-dessous de la peau.

4. Procédé selon l'une quelconque des revendications précédentes, caractérisé en ce que la lumière transmise au tissu est modulée.

5. Procédé selon la revendication 4, caractérisé en ce que l'on utilise différentes fréquences de modulation pour la lumière utilisée pour mesurer la concentration de différents composants et substances.

6. Procédé selon la revendication 5, caractérisé en ce que les différentes fréquences de modulation ne sont pas harmoniques.

7. Procédé selon l'une quelconque des revendications 4 à 6, caractérisé en ce que la modulation de la longueur d'onde de mesure et la modulation de la longueur d'onde de référence ne sont pas en phase.

8. Procédé selon l'une quelconque des revendications 4-7, caractérisé en ce que la modulation s'effectue en interrompant la lumière.

9. Procédé selon l'une quelconque des revendications précédentes, caractérisé en ce que la substance dont on mesure la concentration est le glucose.

10. Procédé selon l'une quelconque des revendications précédentes, caractérisé en ce que la substance dont on mesure la concentration est le CO₂.

11. Procédé selon l'une quelconque des revendications précédentes, caractérisé en ce que la substance dont on mesure la concentration est l'éthanol.

12. Procédé selon l'une quelconque des revendications précédentes, caractérisé en ce que la longueur d'onde d'échantillon et la longueur d'onde de référence utilisées pour le glucose sont 9,5 ± 0,5 »m et 7,7 ± 0,5 »m respectivement.

13. Procédé selon l'une quelconque des revendications 1-11, caractérisé en ce que la longueur d'onde d'échantillon et la longueur d'onde de référence utilisées pour le glucose sont 3,47 ± 0,5 »m et 2,96 ± 0,5 »m respectivement.

14. Procédé selon l'une quelconque des revendications précédentes, caractérisé en ce que la longueur d'onde d'échantillon et la longueur d'onde de référence pour les lipides sont 5,74 ± 0,5 »m et 5,58 ± 0,5 »m respectivement.

15. Procédé selon l'une quelconque des revendications 1-13, caractérisé en ce que la longueur d'onde d'échantillon et la longueur d'onde de référence pour les lipides sont 3,513 ± 0,5 »m et 3,57 ± 0,5 »m respectivement.

16. Procédé selon l'une quelconque des revendications précédentes, caractérisé en ce que la longueur d'onde d'échantillon et la longueur d'onde de référence pour les protéines sont 6,5 ± 0,5 »m et 6,7 ± 0,5 »m respectivement.

17. Appareil pour mesurer in vivo la concentration d'une substance, notamment du glucose, dans le sang malgré des concentrations variables de composants d'interférence tels que les protéines et les lipides, en mesurant l'absorption de longueurs d'ondes sélectionnées de lumière infrarouge dans la plage 1- 40 »m transmise au tissu d'une personne dont on teste le sang pour déterminer sa teneur en la substance, au moins l'une des longueurs d'ondes étant sélectionnée à partir de la plage 3 - 10 »m comprenant
les moyen pour transmettre pour chacune des substances et composants à mesurer deux longueurs d'ondes différentes de lumière infrarouge, l'une étant une longueur d'onde de mesure à laquelle le composant ou la substance à mesurer présente une absorption spécifique et l'autre étant une longueur d'onde de référence à laquelle le composant ou la substance à mesurer présente une faible absorption,
des moyens pour mesurer pour chaque longueur d'onde transmise la radiation absorbée par le tissu en utilisant un détecteur placé dans ou sur la peau et fournissant un signal électrique qui est une expression de la radiation absorbée,
un ordinateur pour calculer les concentrations des composants d'interférence, et
pour calculer la concentration de la substance en utilisant les mesures d'absorption au niveau de la longueur d'onde de référence et de la longueur d'onde mesurant la substance en prenant en compte l'absorption d'interférence provoquée par les composants d'interférence aux longueurs d'ondes de mesure et de référence pour cette substance, l'absorption d'interférence étant calculée en prenant en considération les concentrations mesurées des composants d'interférence et les constantes dérivées expérimentalement.

18. Appareil selon la revendication 17, caractérisé en ce que l'appareil de lumière comprend une source de lumière à bande large et un certain nombre de filtres, chaque filtre transmettant l'une des bandes de lumière étroite sélectionnée.

19. Appareil selon la revendication 17, caractérisé en ce que l'appareil de lumière comprend un certain nombre de diodes électroluminescentes de surface, chaque diode émettant une lumière infrarouge d'une longueur d'onde choisie.

20. Appareil selon la revendication 17, 18 ou 19, caractérisé en ce que l'appareil de lumière comprend des moyens pour focaliser la lumière sur un moyen de couplage optique pour les moyens de transmission de lumière.

21. Appareil selon l'une quelconque des revendications 17-20, caractérisé en ce que l'appareil de lumière comprend un moyen d'interruption destiné à interrompre à des fréquences d'interruption prédéterminées les bandes d'onde lumineuses transmises par l'appareil de lumière.

22. Appareil selon la revendication 21, caractérisé en ce que le moyen d'interruption ou obturateur est un diaphragme rotatif ou oscillant avec des découpes.

23. Appareil selon les revendications 19 et 21, caractérisé en ce que l'obturateur est un circuit électronique commandant l'excitation des diodes électroluminescentes ou des sources à semi-conducteurs.

24. Appareil selon l'une quelconque des revendications précédentes, caractérisé en ce que le détecteur comprend une cellule ATR à sonde à aiguille optique comportant une libre d'entrée et une libre de sortie et sur la libre de sortie un détecteur de lumière fournissant un signal de sortie représentant par un signal électrique l'absorption lumineuse mesurée.

25. Appareil selon l'une quelconque des revendications 17-23, caractérisé en ce que le détecteur comprend une paire de chambres comportant un côté ouvert placé contre la peau à l'emplacement de mesure, et un transducteur de pression mesurant la différence de pression entre les chambres présentant cette pression dilférentielle par un signal électrique.

26. Appareil selon la revendication 25, caractérisé en ce que la chambre sur le côté opposé au côté ouvert possède une entrée pour la radiation infrarouge transmise par l'appareil de lumière.

27. Appareil selon la revendication 25, caractérisé en ce que les deux chambres ont sur leur côté opposé au côté ouvert des entrées pour la radiation infrarouge transmise par l'appareil de lumière.

28. Appareil selon la revendication 27, caractérisé en ce qu'un transducteur supplémentaire est ajouté à chaque chambre aux extrémités de celles-ci et avec son diaphragme situé dans un plan parallèle au plan du diaphragme du transducteur entre les chambres.

29. Appareil selon l'une quelconque des revendications 25-28, caractérisé en ce que chaque chambre comportant une entrée pour la radiation infrarouge communique avec un compartiment contenant un produit dessiccateur.

30. Appareil selon l'une quelconque des revendications 25-28, caractérisé en ce que chaque chambre comportant une entrée pour la radiation infrarouge possède une paroi avec une membrane rigide perméable à l'eau.

31. Appareil selon la revendication 24, caractérisé en ce que la cellule de la sonde à aiguille ATR est revêtue d'un métal de telle sorte qu'elle agit comme dispositif de résonnance plasmatique de surface.

32. Appareil selon la revendication 31, caractérisé en ce que la sonde à aiguille ATR revêtue de métal peut être chemisée à l'aide d'une membrane perméable à la cellulose avec un espace entre la membrane et la sonde.

33. Appareil selon la revendication 32, caractérisé en ce que la membrane peut comporter une enzyme spécifique au glucose ou un système d'enzyme et la sonde ATR utilisée pour mesurer le produit de la réaction enzymatique de la molécule de glucose.

34. Appareil selon les revendications 17-23, caractérisé en ce que le détecteur comprend un dispositif optique avec les libres optiques entrée et sortie et avec un élément optique comprenant un espace d'échantillon pour un échantillon de fluide et une mesure de transmission réalisée sur cet échantillon.
